# EUROPÄISCHE PATENTANMELDUNG

(11) **EP 0 838 455 A1**
(43) Veröffentlichungstag der Anmeldung: **29.04.1998**
(21) Anmeldenummer: 97118718.2
(22) Anmeldetag: 28.10.1997
(51) Int. Cl.: C07D 211/58

(54) **Verfahren zur Herstellung von Bis-(2,2,6,6-tetramethyl-4-piperidyl)-amin**

(30) Priorität: 28.10.1996 DE 19644771
(71) Anmelder: BASF AKTIENGESELLSCHAFT, 67056 Ludwigshafen (DE)
(72) Erfinder: Julius, Manfred, Dr., 67117 Limburgerhof (DE)
(74) Vertreter: Isenbruck, Günter, Dr.

(57) **Zusammenfassung**

Zur Verfügung gestellt wird ein Verfahren zur Herstellung von Bis-(2,2,6,6-tetramethyl-4-piperidyl)-amin der Formel II 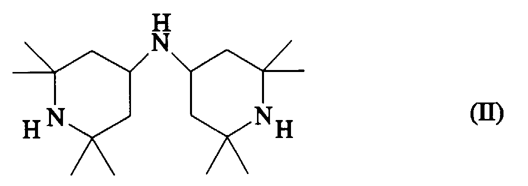 wobei man:
- 2,2,6,6-Tetramethyl-piperidin-4-on (TAA) mit 4-Amino-2,2,6,6-tetramethyl-piperidin (TAD) zu 2,2,6,6-Tetramethyl-4-[(2,2,6,6-tetramethyl-4-piperidyliden)amino]-piperidin (Imin I) der nachfolgenden Formel I umsetzt und 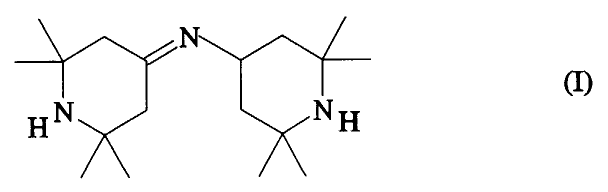
- das 2,2,6,6-Tetramethyl-4-[(2,2,6,6-tetramethyl-4-piperyliden)amino]-piperidin (Imin I) in Gegenwart eines Hydrierkatalysators mit Wasserstoff reagieren läßt.

## Beschreibung

Die Erfindung betrifft ein Verfahren zur Herstellung von Bis-(2,2,6,6-tetramethyl-4-piperidyl)-amin der nachfolgenden Formel II.

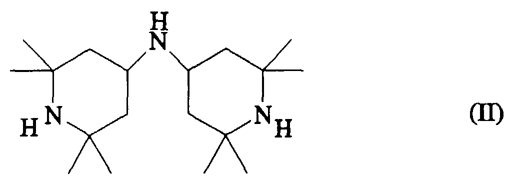

Bis-(2,2,6,6-tetramethyl-4-piperidyl)-amin gemäß der vorstehend genannten Formel II - nachfolgend auch als Amin II abgekürzt - ist ein wichtiges Zwischenprodukt für die Herstellung zahlreicher Lichtstabilisatoren. Es ist nämlich bekannt, daß synthetische Polymere sich unter dem Einfluß von Sonnenlicht oder anderen Quellen ultravioletter Strahlung mehr oder weniger stark chemisch oder physikalisch ändern. Um die nachteilige Wirkung der ultravioletten Strahlung auf synthetische Polymere zu verhindern beziehungsweise zu verzögern, werden den Polymeren geeignete Lichtstabilisatoren zugesetzt. Für die Herstellung dieser Stabilisatoren ist das Amin II ein wichtiges Zwischenprodukt.

Üblicherweise erfolgt die Herstellung des Amins II durch aminierende Hydrierung von geschmolzenem 2,2,6,6-Tetramethylpiperidin-4-on (TAA) in Gegenwart von 4-Amino-2,2,6,6-tetramethyl-piperidin (TAD) an einem Hydrierkatalysator (EP-B 0 302 020). TAA reagiert jedoch empfindlich auf O₂ bzw. Luft, was zu farbgebenden Verunreinigungen führt. Diese finden sich nach Durchführung des erwähnten Verfahrens in den Produkten wieder oder müssen zur Herstellung der Lichstabilisatoren abgetrennt werden.

Gemäß EP-A 0 336 895 erfolgt die Herstellung des Amins II durch reduktive Aminierung von 2,2,6,6-Tetramethyl-4-piperidon (TAA) in Gegenwart eines Hydrierkatalysators und eines sauren Co-Katalysators, wobei die reduktive Aminierung unter Anwendung eines Unterschusses an Ammoniak, bezogen auf TAA, erfolgt.

Die DE-A 33 21 332 beschreibt ein Verfahren zur Herstellung von Polyalkylpiperidylaminen durch reduktive Alkylierung von TAD mit Alkoholen in Gegenwart von Wasserstoff und Hydrierkatalysatoren. Bis-(2,2,6,6-tetramethyl-4-piperidyl)-amin fällt bei diesem Verfahren nur in sehr untergeordnetem Maße als Nebenprodukt an.

EP-A 0 354 184 offenbart ein Verfahren zur Hydrierung von Enaminen-Ketiminen, die durch Umsetzung von TAA mit bestimmten Aminen hergestellt werden.

Die vorstehend genannten Verfahren sind insbesondere wegen der auftretenden farbgebenden Verunreinigungen noch verbesserungsbedürftig.

Aufgabe der Erfindung ist es deshalb, ein alternatives Verfahren zur Herstellung des Amins II bereitzustellen, das einerseits mit wenig apparativem Aufwand und zugleich kostengünstig durchgeführt werden kann und andererseits zu einem hochreinen Produkt mit wenig farbgebenden Verunreinigungen führt. Zusätzlich soll es auch eine gute Selektivität bezüglich des herzustellenden Amins II aufweisen.

Diese Aufgabe wird durch ein Verfahren zur Herstellung von Bis-(2,2,6,6-tetramethyl-4-piperidyl)-amin der Formel II 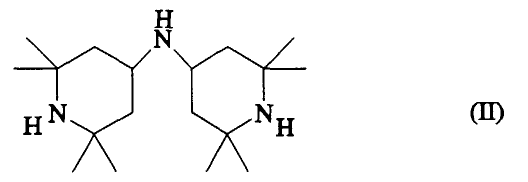 gelöst, wobei man:
2,2,6,6-Tetramethyl-4-[(2,2,6,6-tetramethyl-4-piperidyliden)amino]-piperidin (Imin I) der nachfolgenden Formel I 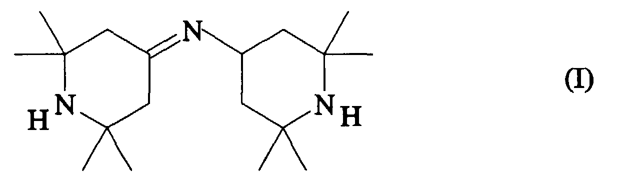 in Gegenwart eines Hydrierkatalysators mit Wasserstoff reagieren läßt.

In einer Ausführungsform wird - ausgehend von dem Imin I - dasselbe direkt dem vorstehend genannten Hydrierungsschritt unterzogen und so das Amin II hergestellt.

Bevorzugt erfolgt die Umsetzung des Imins I - d.h. die Hydrierung - bei einer Temperatur im Bereich von 20°C bis 150°C. Der Druck liegt bevorzugterweise im Bereich von 1 bis 300 bar.

Bei einer weiteren Ausführungsform des erfindungsgemäßen Verfahrens wird zunächst das Imin 1 durch Umsetzung von 2,2,6,6-Tetramethyl-piperidin-4-on (TAA) mit 4-Amino-2,2,6,6-tetramethyl-piperidin (TAD) unter Freisetzung von Wasser in einem ersten Verfahrensschritt gemäß folgendem Schema hergestellt:

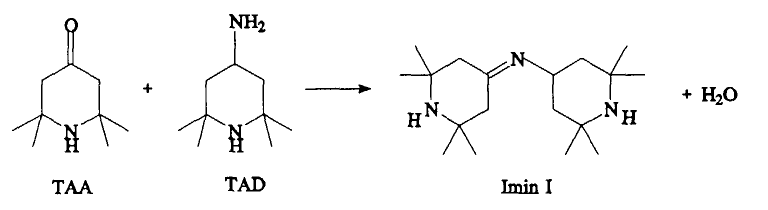

Die mit der vorstehenden Reaktionsgleichung beschriebene Kondensation von TAA mit TAD zu dem Imin I wird bevorzugt nicht katalysiert sowie lösungsmittelfrei bei Temperaturen von 30 bis 90°C durchgeführt. Das Produkt Imin 1 wird durch Kristallisation gewonnen [Umsatz z.B. 75,8 %, Selektivität 95,6 % nach GC].

Die zur Herstellung des Amins II durchzuführende Hydrierung des Imins mit der Formel I kann diskontinuierlich im Autoklaven mit einem handelsüblichen Suspensions- oder Festbettkatalysator oder kontinuierlich im Rohrreaktor an einem Festbettkatalysator durchgeführt werden. Sie erfolgt gemäß der nachfolgenden Reaktionsgleichung:

Bei dieser Ausführungsform des erfindungsgemäßen Verfahrens liegt die Selektivität im zweiten Verfahrensschritt bei etwa 70 %; der Umsatz, 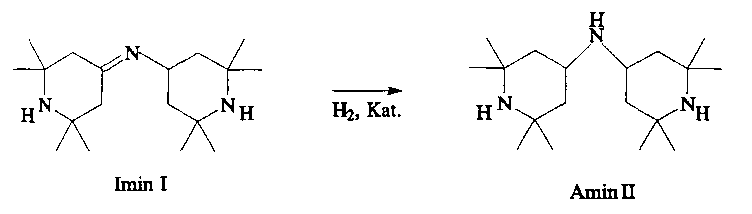 bezogen auf das als Ausgangsprodukt dienende Imin I, liegt dabei bei über 90 %.

Das als Nebenprodukt gebildete 4-Amino-2,2,6,6-tetramethylpiperidin (TAD) kann seinerseits wieder für die Synthese des Ausgangsstoffs Imin I eingesetzt werden. Die beiden Verfahrensschritte können direkt nacheinander ausgeführt werden. Bevorzugt ist jedoch eine dazwischerliegende Isolierung und Reinigung des Imins.

Nachfolgend wird das erfindungsgemäße Verfahren durch zwei Beispiele erläutert:

### Beispiel

### I. Synthese von 2,2,6,6-Tetramethyl-4-[(2,2,6,6-tetramethyl-4-piperidyliden)amino]-piperidin (Imin I)

In einem Rührkolben wurden unter N₂ 85,95 g (0,55 Mol) TAD und 77,62 g (0,50 Mol) TAA vorgelegt und 4 h lang auf 50°C erhitzt. Nach GC besaß die Reaktionsmischung danach folgende Zusammensetzung (Fl.-%): 15,76 % TAA, 8,47 % TAD, 72,46 % Imin I, 3,31% Rest (GC-Bedingungen: Kapillarsäule 30 m RTX-5 Amine; 50 - 250°C, 5°C/Min).

Dies entspricht einer Selektivität für die Bildung des Imins von 95,6 % bei einem Umsatz von 75,8 %.

Das reine, weiße Imin wurde durch Kristallisation aus Petrolether 30/75 gewonnen. Smp. 59 - 61 °C.

| **Elementaranalyse:** | | | | |
|---|---|---|---|---|
| | C | H | N | |
| % berechnet | 73,66 | 12,02 | 14,32 | C₁₈H₃₅N₃ |
| % gefunden | 73,3 | 12,1 | 14,2 | (MG = 293,50) |

¹³C-NMR (62,9 MHz, CDCl₃):
δ = 28,76 (2·CH₃), 31,45 (2·CH₃), 31,87 (2·CH₃), 35,06 (2·CH₃), 42,11 (CH₂-CH), 46,05 (2·CH₂-C=N), 50,51 (2·C(CH₃)₂), 51,40 (C(CH₃)₂), 52,26 (C(CH₃)₂), 53,72 (CH-N=C), 54,07 (CH₂-CH), 168,28 (C=N).

- Massenspektrum (EI):: M⁺ = 293.
- IR-Spektrum:: 1650 cm⁻¹ (C=N).

### II. Synthese von Bis(2,2,6,6-tetramethyl-4-piperidyl)amin (Amin II)

In einem RA4-Autoklaven mit Begasungsrührer wurde eine Lösung von 4,0 g (13,6 mMol) 2,2,6,6-Tetramethyl-4-[(2,2,6,6-tetramethyl-4-piperidyliden)amino]-piperidin (Imin I) (Reinheit nach GC: 96,3 %) in trockenem Methanol zusammen mit 2,0 g Raney-Nickel vorgelegt. Nach dem Inertisieren mit N₂ wurde mit Wasserstoff ein Druck von 20 bar eingestellt, unter Rühren auf 90°C erhitzt und der Wasserstoffdruck auf 100 bar erhöht. Nach 8 h kühlte man ab, entspannte und analysierte die Reaktionslösung per GC (Fl. - %) und GC-MS (EI). Es ergab sich folgende Zusammensetzung (ohne Lösungsmittel):

| | |
|---|---|
| Bis-(2,2,6,6-tetramethyl-4-piperidyl)-amin: | 68,2 % |
| Triacetondiamin (TAD): | 14,2 % |
| Triacetonaminoalkohol (TAA-ol): | 3,1 % |
| 2,2,6,6-Tetramethylpiperidin (TMP): | 1,7 % |
| Imin I: | 0,5 % |
| Rest: | 12,3 % |

Der Umsatz: an Imin I betrug 99,5 % bei einer Selektivität für die Bildung Von II von 71,1 %. Die Selektivität für die Bildung von TAD betrug 14,8 %.

## Patentansprüche

1. Verfahren zur Herstellung von Bis-(2,2,6,6-tetramethyl-4-piperidyl)-amin der Formel II 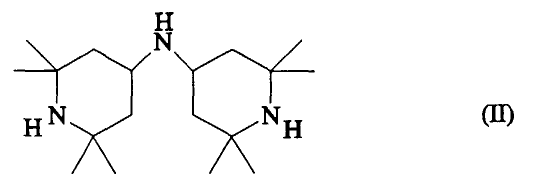 dadurch gekennzeichnet, daß man 2,2,6,6-Tetramethyl-4-[(2,2,6,6-tetramethyl-4-piperidyliden)amino]-piperidin (Imin I) der nachfolgenden Formel I 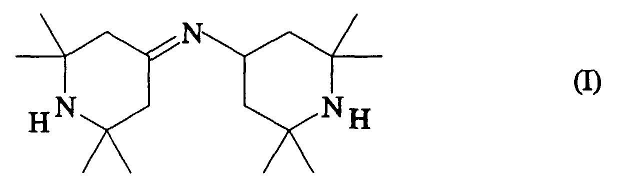 in Gegenwart eines Hydrierkatalysators mit Wasserstoff reagieren läßt.

2. Verfahren nach Anspruch 1, dadurch gekennzeichnet, daß die Umsetzung des 2,2,6,6-Tetramethyl-4-[(2,2,6,6-tetramethyl-4-piperidyliden)amino]-piperidins der Formel I (Imin I) bei Temperaturen im Bereich von 20°C bis 150°C und bei einem Druck im Bereich von 1 bis 300 bar erfolgt.

3. Verfahren nach einem der Ansprüche 1 oder 2, dadurch gekennzeichnet, daß man das Imin I durch Umsetzung von 2,2,6,6-Tetramethyl-piperidin-4-on (TAA) mit 4-Amino-2,2,6,6-tetramethyl-piperidin (TAD) herstellt.
